Europäisches Patentamt

(19) European Patent Office  (11) Numéro de publication: **0 179 695**

Office européen des brevets  **B1**

(12) **FASCICULE DE BREVET EUROPÉEN**

(45) Date de publication du fascicule du brevet: **29.03.89**  (51) Int. Cl.⁴: **A 61 F 2/44**

(21) Numéro de dépôt: **85401869.4**

(22) Date de dépôt: **25.09.85**

(54) Prothèse vertébrale, en particulier pour vertèbres cervicales.

(30) Priorité: **26.09.84 FR 8414791**

(43) Date de publication de la demande: **30.04.86 Bulletin 86/18**

(45) Mention de la délivrance du brevet: **29.03.89 Bulletin 89/13**

(84) Etats contractants désignés: **AT BE CH DE GB IT LI LU NL SE**

(56) Documents cité:
**DE-A-2 547 816**
**GB-A-2 083 754**
**US-A-3 426 364**
**US-A-3 486 505**

(73) Titulaire: **Kehr, Pierre, 25 Rue Schweighaeuser, F-67000 Strasbourg (FR)**

(72) Inventeur: **Kehr, Pierre, 25 Rue Schweighaeuser, F-67000 Strasbourg (FR)**

(74) Mandataire: **CABINET BONNET- THIRION, 95 Boulevard Beaumarchais, F-75003 Paris (FR)**

EP 0 179 695 B1

## Description

La présente invention concerne d'une manière générale les prothèses vertébrales, et elle vise plus particulièrement, mais non exclusivement, celles susceptibles d'être appliquées aux vertèbres cervicales.

Ainsi qu'on le sait, par suite d'une dégénérescence naturelle due au vieillissement, ou d'un traumatisme résultant par exemple d'une pratique sportive ou d'un accident, les vertèbres cervicales peuvent être le siège d'une situation pathologique conduisant un ou plusieurs des disques vertébraux qui les séparent deux à deux à presser, plus ou moins fermement, à l'arrière, dans le canal médullaire, la moelle épinière et le réseau sensitif correspondante.

Il y a dès lors ce qu'il est convenu d'appeler une "hernie discale" ou bien un "ostéophyte", qu'il faut traiter.

Pour ce faire, le praticien doit intervenir par l'avant, l'accès par l'arrière se trouvant occulté par la moelle épinière.

Autrement dit, pour avoir accès à la lésion discale à traiter, le praticien doit passer à travers le disque vertébral concerné, et donc l'éliminer.

Dans la plupart des cas, et c'est le cas pour les vertèbres cervicales, il faut remplacer par un greffon le disque vertébral ainsi éliminé.

La retenue élastique développée par les vertèbres qu'il faut écarter pour sa mise en place suffit parfois au maintien naturel d'un tel greffon, avant la réossification de l'ensemble.

Mais, le plus souvent, notamment lorsque deux niveaux distincts sont à traiter, c'est-à-dire lorsque l'intervention concerne non pas un seul disque vertébral mais deux, ou lorsque cette intervention est due à un traumatisme relativement important, il faut procéder à la mise en place d'une prothèse pour le maintien de ce greffon.

Il s'agit, en pratique, à ce jour, d'une simple plaquette, en acier par exemple, propre à être rapportée, par l'avant, sur deux vertèbres distinctes, par exemple par des vis.

L'expérience montre qu'une telle plaquette est par elle-même bien tolérée, et qu'il n'est que très rarement nécessaire de la retirer par la suite.

A ce jour, le greffon mis en oeuvre est un greffon naturel, et il est usuellement prélevé sur la tranche de l'os iliaque du patient à traiter.

Par sa zone périphérique, corticale, et donc dure, qui est en forme de fer à cheval, un tel greffon présente la dureté et la résistance mécanique nécessaire, tandis que, par sa zone centrale, qui est spongieuse, il est avantageusement très facilement réhabitable par un bourgeonnement osseux, les plateaux vertébraux qui l'encadrent ayant été par ailleurs légèrement avivés lors de l'intervention pour stimuler la formation de cellules osseuses et ainsi favoriser l'ostéogenèse recherchée pour la prise de l'ensemble.

Cependant, la mise en oeuvre, ainsi, d'un greffon naturel, conduit à des inconvénients, sinon majeurs, au moins non négligeables.

Tout d'abord, son prélèvement sur le patient impose une intervention supplémentaire à celui-ci, et l'expérience montre que, en raison notamment de la masse musculaire inévitablement concernée par cette dernière, le temps global de récupération pour le patient se trouve fréquemment singulièrement alourdi.

En outre, la prise d'un tel greffon naturel est toujours relativement longue, et elle peut imposer au patient le port d'un plâtre ou d'une minerve pendant de nombreuses semaines.

Enfin, cette prise, qui n'est jamais acquise, peut être déficiente.

Dans la demande de brevet anglais n° 2 083 754, il est proposé par ailleurs une prothèse de type vérin, qui se substituant à un disque vertébral en s'arc-boutant entre les deux disques vertébraux qui l'encadrent, a pour but de stabiliser la colonne, mais qui, même s'il peut faire ultérieurement l'objet d'un certain enrochement osseux, ne comporte pas lui-même aucun élément de greffe, c'est-à-dire aucun élément susceptible de favoriser le bourgeonnement osseux nécessaire à un tel enrochement.

La présente invention a d'une manière générale pour objet une prothèse vertébrale susceptible de minimiser, sinon annuler, ces inconvénients.

D'une manière plus précise, elle a pour objet une prothèse vertébrale comportant une plaquette propre à être rapportée, par exemple par des vis, sur deux vertèbres distinctes, cette prothèse vertébrale étant caractérisée d'une manière générale en ce que, dans sa zone médiane, ladite plaquette porte transversalement par avance un greffon artificiel fait d'une manière propre à favoriser un bourgeonnement osseux et présentant à sa surface des logements propres à favoriser un tel bourgeonnement osseux.

Ainsi, cette prothèse vertébrale, qui convient aussi bien au cas où un seul niveau est à traiter qu'au cas où deux niveaux distincts nécessitent une intervention, sa hauteur, et celle du greffon artificiel qu'elle comporte, étant alors prévues en conséquence, conserve les avantages dus à la présence d'une plaquette de maintien, tout en éliminant les inconvénients usuellement attachés, tel que rappelé ci-dessus, à la mise en oeuvre d'un greffon naturel.

Il s'avère en effet que, indépendamment du fait que toute intervention spécifique de prélèvement est évitée, un tel greffon artificiel est avantageusement susceptible de conduire à une reduction notable du temps de port éventuel d'un plâtre ou d'une minerve, aucun inconvénient majeur n'étant par exemple à craindre en cas de prise plus ou moins différée.

La récupération d'ensemble du patient est donc avantageusement plus réduite et plus sûre.

Les caractéristiques et avantages de l'invention ressortiront d'ailleurs de la description qui va suivre, à titre d'exemple, en référence aux dessins schématiques annexés sur lesquels:

la figure 1 est une vue en perspective d'une prothèse vertébrale suivant l'invention;

la figure 2 en est une vue en coupe axiale, suivant la ligne II - II de la figure 1;

la figure 3 en est une vue en coupe transversale, suivant la ligne III - III de la figure 1;

la figure 4 est une vue qui, analogue à celle de la figure 2, illustre, à échelle inférieure, la mise en place de la prothèse suivant l'invention entre deux vertèbres cervicales;

les figures 5 et 6 sont des vues en coupe transversale, qui, analogues à celle de la figure 3, concernent chacune respectivement une variante de réalisation.

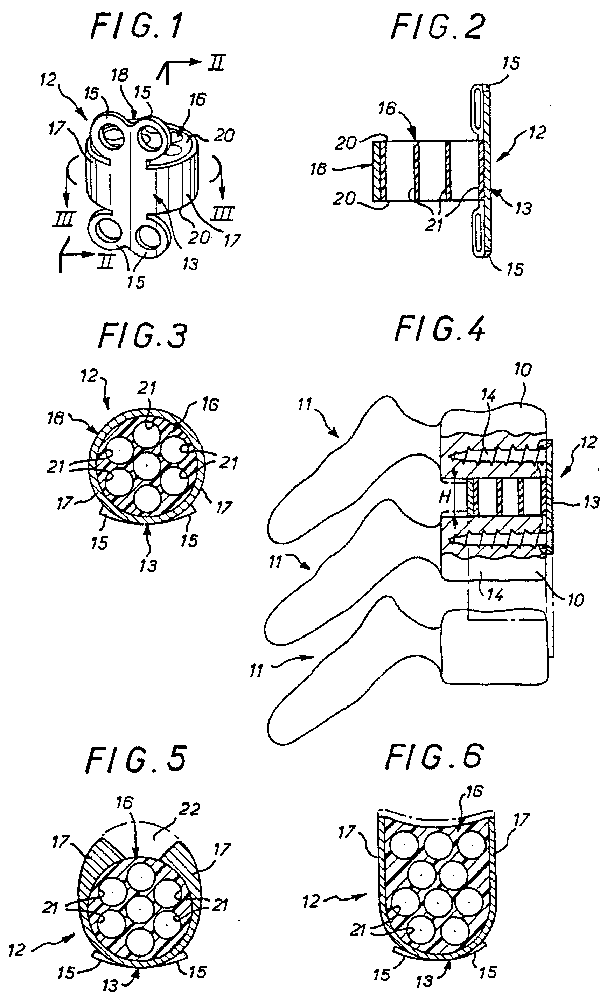

Ces figures illustrent, à titre d'exemple, en trait plein, le cas ou seul un niveau est à traiter, entre les plateaux vertébraux 10 de deux vertèbres 11 successives, tel que schématisé en traits fins sur la figure 4.

La prothèse vertébrale 12 mise en oeuvre à cet effet suivant l'invention comporte, de manière connue en soi, une plaquette 13 propre à être rapportée, par exemple par des vis 14, tel que représenté, sur les deux vertèbres 11 concernées, et, plus précisément, sur la face avant de leurs plateaux vertébaux 10.

Dans la forme de réalisation représentée, la plaquette 13 présente à cet effet, à chacune de ses extrémités, deux oreilles 15, propres chacune au passage d'une telle vis 14.

Ces oreilles 15, qui sont jumelées, sont globalement cintrées, à l'image de la face avant du plateau vertébral 10 d'une vertèbre 11.

Suivant l'invention, la plaquette 13 porte transversalement, par avance, un greffon artificiel 16.

En pratique, et tel que représenté, ce greffon artificiel 16 est au moins partiellement enserré, sur une partie au moins de son périmètre, par deux bras 17 solidaires de la plaquette 13 qui le porte.

Dans la forme de réalisation plus particulièrement représentée sur les figures 1 à 4, ces bras 17 se rejoignent l'un l'autre, et forment ainsi conjointement une enveloppe 18 qui enserre le greffon artificiel 16 sur la totalité de son périmètre.

Plus précisément, dans cette forme de réalisation, cette enveloppe 18 est tubulaire, elle a en section un contour circulaire, et elle est d'un seul tenant avec la plaquette 13, l'ensemble des bras 17 qu'elle forme et du greffon artificiel 16 qu'elle enserre s'étendant conjointement en porte-à-faux à compter de la zone médiane de ladite plaquette 13, entre les oreilles 15 de celle-ci, à la manière d'une console.

En pratique, la plaquette 13 et l'enveloppe 18 sont en métal, et par exemple en acier inoxydable ou en titane.

L'ensemble peut par exemple être obtenu par découpe appropriée d'un tronçon de tube d'un tel métal ou alliage, ou être obtenu par découpe et pliage appropriés d'un flan initialement plat de celui-ci, les bras 17 ayant alors leurs extrémités, convenablement affrontées l'une à l'autre par cintrage, éventuellement solidarisées l'une à l'autre, par exemple par soudage.

Mais il va de soi que tout autre mode de fabrication peut convenir.

Le greffon artificiel 16, quant à lui, fait d'une matière propre à favoriser le bourgeonnement osseux, est de préférence en céramique, et par exemple en alumine frittée.

Il s'avère, en effet, que, en milieu osseux, une telle matière, qui est par ailleurs bien tolérée, présente par elle-même une certaine agressivité de nature à en favoriser l'enrochement.

En pratique, le greffon artificiel 16 se présente, dans la forme de réalisation représentée, sous la forme d'une tronçon de cylindre, qui affleure à la surface de l'enveloppe 18 qui l'enserre, et dont les surfaces transversales correspondantes 20 sont, à l'image de celles de celle-ci, globalement planes, perpendiculairement à la direction d'allongement de la plaquette 13.

De préférence, le greffon artificiel 16 présente, en creux, sur chacune de ses surfaces transversales 20, des logements 21 propres à favoriser un bourgeonnement osseux.

En pratique, et tel que représenté, ces logements 21 s'étendent en tubes de l'une à l'autre des surfaces transversales 20 du greffon artificiel 16.

Ils constituent ainsi avantageusement, lorsqu'ils sont envahis par le bourgeonnement osseux résultant de l'ostéogenèse recherchée, un guide favorable à une prolifération en ligne de ce bourgeonnement osseux.

De préférence, le greffon artificiel 16 ainsi constitué est brut d'usinage.

Il présente donc, tant sur ses surfaces transversales 20 qu'à l'intérieur de ses logements 21, un grain de surf... négligeable, compris entre 1 à 50 micro... ...ple, favorable à un bon accrochage mec... ... bourgeonnement osseux qui doit l'envahir.

En pratique, ce greffon artificiel 16 est simplement engagé à force dans l'enveloppe 18 qui l'enserre.

Pour faciliter un tel engagement, l'ensemble que constitue cette enveloppe 18 et la plaquette 13 qui la porte peut être préalablement chauffé, et il en résulte, au refroidissement, au moins dans une certaine mesure, un frettage plus ou moins prononcé du greffon artificiel 16, favorable à la tenue de celui-ci.

Mais un tel frettage n'est pas impératif.

En outre, lorsque seuls de simples bras 17 distincts sont mis en oeuvre, ces bras 17 sont simplement refermés, sans necessairement se rejoindre, sur le greffon artificiel 16 qu'ils doivent enserrer.

La mise en place de la prothèse vertébrale 12 suivant l'invention se fait suivant un processus usuel.

Après avoir préparé l'implantation des vis 14 sur les vertèbres 11 concernées, et avoir avivé les faces concernées de leur plateau vertébral 10, tel que schématisé en traits interrompus à la figure 4, en correspondance avec la hauteur H du greffon artificiel 16, la prothèse vertébrale 12 suivant l'invention est engagée, par ce greffon artificiel 16, entre les plateaux vertébraux 10 ainsi avivés, jusqu'à contact de sa plaquette 13 contre la face avant de ceux-ci, et les vis 14 sont alors à leur tour mises en place.

Dans ce qui précède, il a été supposé que, comme indiqué, seul un niveau est à traiter.

Mais, tel que schématisé en traits interrompus à la figure 4, dans le cas où deux niveaux necessitent simultanément une intervention, soit parce que deux disques adjacents sont atteints par un processus pathologique, soit parce que tout le plateau ou corps vertébral compris ces deux disques doit être remplacé, comme cela se voit dans les destructions cancereuses ou dans les fractures graves, la hauteur H du greffon artificiel 16 est choisie en conséquence, et donc celle de l'ensemble de la prothèse vertébrale 12 mise en oeuvre, avec elimination du plateau vertébral 10 intermédiaire.

Dans la variante de réalisation représentée sur la figure 5, le greffon artificiel 16 conserve extérieurement un contour circulaire, mais les bras 17 n'ayant pas une épaisseur constante, il se trouve excentré par rapport au contour extérieur de ces bras 17.

En outre, et tel que représenté en trait plein sur cette figure 5, les bras 17 peuvent ne pas se rejoindre l'un l'autre, une fente 22 subsistant alors entre eux en service.

Mais, tel que schématisé en traits interrompus, ils peuvent aussi bien se rejoindre l'un l'autre, comme précédemment.

Dans la variante de réalisation illustrée par la figure 6, une forme en fer à cheval, ou en U, analogue à celle d'un greffon artificiel, est conservée.

Tel que représenté en trait plein, les bras 17 peuvent être distincts, et n'enserrer ainsi le greffon artificiel 16 que sur trois côtés.

Mais, comme précédemment, et tel que schématisé en traits interrompus, ce greffon artificiel 16 peut aussi être enserré sur la totalité de son périmètre.

La présente invention ne se limite d'ailleurs pas aux formes de réalisation décrites et représentées, mais englobe toute variante d'exécution et/ou de combinaison de leurs divers éléments, le cadre de l'invention étant defini pas les revendications.

En particulier, les bras enserrant le greffon artificiel mis en oeuvre ne sont pas nécessairement d'un seul avec la plaquette qui les porte.

Ils peuvent aussi bien être rapportés de toute manière appropriée sur cette plaquette, par exemple par soudage.

De plus, au lieu d'être elles aussi d'un seul tenant avec cette plaquette, les oreilles que présente celle-ci pour le passage d'un moyen de fixation peuvent être montées ajustables en position sur une telle plaquette.

L'ouverture que présentent de telles oreilles pour le passage d'un tel moyen de fixation peut en outre, au lieu d'être circulaire, comme plus particulièrement représenté, être allongée en boutonnière, pour permettre également, au moins dans une certaine mesure, un ajustement en position de l'ensemble.

Corollairement, les logements que présente en creux le greffon artificiel mis en oeuvre ne forment pas nécessairement des tubes d'une des surfaces transversales à l'autre de celui-ci, bien que cette disposition soit préférée, et/ou les dites surfaces transversales ne sont pas nécessairement planes.

Pour le traitement d'un même niveau, deux demi-greffons peuvent d'ailleurs être mis en oeuvre, dos à dos, en étant par exemple enchâssés dans une même console, l'un pour contact avec le plateau vertébral supérieur du niveau concerné, l'autre pour contact avec le plateau inférieur de ce même niveau.

Dans le cas où des dispositions propres à un ajustement en position sont adoptées, deux greffons distincts, convenablement étagés de manière réglable, peuvent être mis en oeuvre, pour le traitement de deux niveaux différents.

Enfin, le domaine d'application de l'invention ne se limite pas à celui des seules vertèbres cervicales mais s'étend au contraire également aussi bien à celui des vertèbres dorsales ou lombaires.

**Revendications**

1. Prothèse vertébrale comportant une plaquette (13) propre à être rapportée, par exemple par des vis, sur deux vertèbres distinctes, caractérisée en ce que, dans sa zone médiane, ladite plaquette (13) porte transversalement par avance un greffon artificiel (16) fait d'une matière propre à favoriser un bourgeonnement osseux et présentant à sa surface des logements (21) propres à favoriser un tel bourgeonnement osseux.

2. Prothèse vertébrale suivant la revendication 1, caractérisée en ce que le greffon artificiel (16) est au moins partiellement enserré, sur une partie au moins de son périmètre, par deux bras (17) solidaires de la plaquette (13) qui le porte.

3. Prothèse vertébrale suivant la revendication 2, caractérisée en ce que, se rejoignant l'un l'autre, lesdits bras (17) forment conjointement une enveloppe (18) qui enserre le greffon artificiel (16) sur la totalité de son périmètre.

4. Prothèse vertébrale suivant la revendication 3, caractérisée en ce que ladite enveloppe (18) est tubulaire.

5. Prothèse vertébrale suivant la revendication 4, caractérisée en ce que ladite enveloppe (18) a en section un contour circulaire.

6. Prothèse vertébrale suivant l'une quelconque des revendications 2 à 5, caractérisée en ce que le greffon artificiel (16) et les bras (17) qui l'enserrent s'étendent conjointement en porte-à-faux à compter de la plaquette (13) qui les porte.

7. Prothèse vertébrale suivant l'une quelconque des revendications 2 à 6, caractérisée en ce que les bras (17) qui enserrent le greffon artificiel (16) sont d'un seul tenant avec la plaquette (13).

8. Prothèse vertébrale suivant l'une quelconque des revendications 1 à 7, caractérisée en ce que, la plaquette (13) présentant à ses extrémités des oreilles (15) propres au passage de moyens de fixation tels que des vis, le greffon artificiel (16) s'étend entre lesdites oreilles (15).

9. Prothèse vertébrale suivant l'une quelconque des revendications 1 à 8, caractérisée en ce que les logements (21) du greffon artificiel (16) s'étendent en tubes de l'une à l'autre des surfaces transversales (20) de celui-ci.

10. Prothèse vertébrale suivant l'une quelconque des revendications 1 à 9, caractérisée en ce, que la plaquette (13) est en métal et le greffon artificiel (16) est en alumine frittée.

## Patentansprüche

1. Wirbelprothese mit einer Platte (13), die, beispielsweise durch Schrauben, an zwei gesonderten Wirbelknochen befestigbar ist, dadurch gekennzeichnet, daß die Platte (13) in ihrem mittleren Bereich quer als Vorsprung ein künstliches Transplantat (Implantat) (16) trägt, das aus einem Material, das ein Knochenwachstum begünstigt, hergestellt ist und an seiner Oberfläche Aufnahmeräume (21) aufweist, welche ein solches Einwachsen des Knochens begünstigen.

2. Wirbelprothese nach Anspruch 1, dadurch gekennzeichnet, daß das künstliche Implantat (16) mindestens teilweise über mindestens einen Teil seines Umfangs durch zwei Arme (17) eingespannt ist, die fest mit der Platte (13) verbunden sind, welche das Implantat trägt.

3. Wirbelprothese nach Anspruch 2, dadurch gekennzeichnet, daß die aufeinander treffenden Arme (17) miteinander eine Hülle (18) bilden, welche das künstliche Implantat (16) über seinen gesamten Umfang einspannen.

4. Wirbelprothese nach Anspruch 3, dadurch gekennzeichnet, daß die Hülle (18) rohrförmig ist.

5. Wirbelprothese nach Anspruch 4, dadurch gekennzeichnet, daß die Hülle (18) im Querschnitt einen kreisförmigen Umriß hat.

6. Wirbelprothese nach einem der Ansprüche 2 bis 5, dadurch gekennzeichnet, daß das künstliche Implantat (16) und die dieses einspannenden Arme (17) sich gemeinsam freitragend von der sie tragenden Platte (13) aus erstrecken.

7. Wirbelprothese nach einem der Ansprüche 2 bis 6, dadurch gekennzeichnet, daß die das künstliche Implantat (16) einspannenden Arme (17) einstückig mit der Platte (13) sind.

8. Wirbelprothese nach einem der Ansprüche 1 bis 7, dadurch gekennzeichnet, daß die Platte (13) an ihren Enden Ösen (15) für den Durchtritt von Befestigungsmitteln, wie Schrauben, aufweist und das künstliche Implantat (16) sich zwischen diesen Ösen (15) erstreckt.

9. Wirbelprothese nach einem der Ansprüche 1 bis 8, dadurch gekennzeichnet, daß die Aufnahmeräume (21) des künstlichen Implantats (16) sich rohrförmig von der einen zur anderen der quer verlaufenden Oberflächen (20) desselben erstrecken.

10. Wirbelprothese nach einem der Ansprüche 1 bis 9, dadurch gekennzeichnet, daß die Platte (13) aus Metall besteht und das künstliche Implantat (16) aus gesintertem Aluminiumoxid besteht.

## Claims

1. A vertebral prosthesis comprising a plate portion (13) which is capable of being connected to two separate vertebrae, for example by screws, characterised in that, in its median zone, said plate portion (13) transversely carries in advance an artificial graft (16) made of a material suitable for promoting boney growth and having at its surface accommodation means (21) for promoting such boney growth.

2. A vertebral prosthesis according to claim 1, characterised in that the artificial graft (16) is at least partially embraced over a part at least of its perimeter by two arms (17) which are fixed with respect to the plate portion (13) which carries it.

3. A vertebral prosthesis according to claim 2, characterised in that, joining together, said arms (17) jointly form a casing (18) which embraces the artificial graft (16) over the whole of its perimeter.

4. A vertebral prosthesis according to claim 3, characterised in that said casing (18) is tubular.

5. A vertebral prosthesis according to claim 4, characterised in that said casing (18) is of a section of circular contour.

6. A vertebral prosthesis according to any one of claims 2 to 5, characterised in that the artificial graft (16) and the arms (17) which embrace it extend jointly in cantilever relationship from the plate portion (13) which carries them.

7. A vertebral prosthesis according to any one of claims 2 to 6, characterised in that the arms (17) which embrace the artificial graft (16) are in one piece with the plate portion (13).

8. A vertebral prosthesis according to any one of claims 1 to 7, characterised in that, the plate portion (13) being provided at its ends with lugs (15) for receiving fixing means such as screws, the artificial graft (16) extends between said lugs (15).

9. A vertebral prosthesis according to any one of claims 1 to 8, characterised in that the accommodation means (21) of the artificial graft (16) extend in the form of tubes from one of the transverse surfaces (20) thereof to the other.

10. A vertebral prosthesis according to any one of claims 1 to 9, characterised in that the plate portion (13) is of metal and the artificial graft (16) is of sintered alumina.

# FIG.1

# FIG.2

# FIG.3

# FIG.4

# FIG.5

# FIG.6

1